(19) 

Europäisches
Patentamt
European
Patent Office
Office européen
des brevets

(11) **EP 4 574 019 A1**

(12) **EUROPEAN PATENT APPLICATION**
published in accordance with Art. 153(4) EPC

(43) Date of publication:
25.06.2025 Bulletin 2025/26

(21) Application number: 23854172.6

(22) Date of filing: 18.07.2023

(51) International Patent Classification (IPC):
*A61B 3/113* (2006.01)    *A61B 3/14* (2006.01)

(52) Cooperative Patent Classification (CPC):
A61B 3/113; A61B 3/14; Y02P 90/30

(86) International application number:
PCT/CN2023/107803

(87) International publication number:
WO 2024/037267 (22.02.2024 Gazette 2024/08)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.08.2022 CN 202210989508

(71) Applicant: Shangai Baiyi Healthcare Technology
Co, Ltd
Shanghai 201203 (CN)

(72) Inventors:
• TIAN, Chaonan
Shanghai 201203 (CN)
• WANG, Youxiang
Shanghai 201203 (CN)
• WANG, Youzhi
Shanghai 201203 (CN)
• DU, Dong
Shanghai 201203 (CN)

(74) Representative: Elzaburu S.L.P.
Paseo de la Castellana 259C
Torre de Cristal, planta 28
28046 Madrid (ES)

(54) **METHOD AND SYSTEM FOR EVALUATING DEGREE OF EYEBALL MOVEMENT AND STORAGE MEDIUM**

(57) The application relates to a method, a system and a storage medium for evaluating the degree of eyeball movement. The evaluation method includes: in a near-infrared light field of 700-1200 nm, capturing and acquiring a first eye position image of the user looking straight ahead at a position in front of the eyeball; capturing and acquiring a second eye position image and a third eye position image of the user's eyeball moving to a limit position along the direction to be measured in front of the eyeball; comparing the first eye position image with the second eye position image and the third eye position image respectively, and calculating a movement angle of the degree of eyeball movement. According to the technical scheme of the invention, the eye images are captured in the near-infrared light field, and the iris and pupil are effectively distinguished, so that the pupil edge is accurately identified, and then the angle of eyeball movement is calculated by comparing different eye position images, and the calculation precision of the angle of eyeball movement is further improved through compensation, so that the angle of eyeball movement in each direction to be measured is accurately calculated.

Fig. 1

**Description**

TECHNICAL FIELD

This application belongs to the field of ocular detection technology, and specifically relates to a method, system and storage medium for evaluating degree of eyeball movement.

BACKGROUND

The oculomotor, trochlear and abducent nerves have the function of controlling the movement of the extraocular muscles of the eye, which are called the oculomotor nerves. When the above nerve or nerve nucleus is damaged, alone or together, it can appear in eye movement or as diplopia. Complete damage occurs when the external eye muscles are paralyzed, and the eye is fixed. Extraocular muscle injury, or infection or myopathy caused by extraocular muscle paralysis, can also appear in eyeball movement, clinically collectively referred to as eyeball movement disorders. Eyeball movement disorders may also be associated with medical diseases, including orbital diseases, diabetes, neuroinflammation, etc. Therefore, how to evaluate the eyeball movement capabilities of both eyes is of great significance for detection of eye disorders.

In the prior art, the detection of eyeball movement is usually carried out manually by the doctor. The specific detection methods are as follows: 1) Eyeball movement examination in one eye: cover the opposite eye, start from the first eye position, ask the patient to look at the flashlight, and move along the diagnostic direction of gaze; 2) Eyeball movement examination of both eyes: starting from the first eye position, ask the patient to look at the flashlight and move along the diagnostic direction of gaze. Binocular movement examination can evaluate the relative position of the eyes and obtain information different from the monocular examination. However, the above detection methods, there is no objective data, and cannot record the changes of patients over time, cannot form continuous data.

In addition, the prior art also discloses the use of automatic detection methods to detect eyeball movement. For example, in the Chinese invention patent "An eyeball mobility detector" (application number: 202011260253.4, publication date: February 19, 2021), the head of the patient is fixed through a head fixing frame, an indicator light is arranged in front of the head fixing frame, the indicator light emits visible light beams in the direction away from the eyes of patent, and the visible light beams extend out of static field of vision of the patient, and the imaging lens is arranged in front of the head fixing frame. The imaging lens is used to capture the images of the patient's eyeball when the eyeball rotates in the direction of the visible light beams, and the patient's eyeball movement condition is obtained through the eye image captured by the imaging lens. However, in the above technical scheme, the eye images are not specifically distinguished and processed, resulting in insufficient detection accuracy of eyeball movement condition. For example, in the Chinese patent "A computer-based device and method for detecting eyeball movement distance and binocular movement consistency deviation" (Application No. : 201710054692.1, publication date: June 13, 2017), a corneal limbus extraction unit extracts baseline photos from the camera and the corneal limbus in each test photo, and calculates the movement distance of the eyeball in all directions through extracted points on the corneal limbus, so as to calculate a binocular movement consistency deviation. However, since the corneal limbus is the transitional zone between the cornea and the sclera, the extracted boundary of the corneal limbus is not sufficiently clear, which affects the accuracy of the final result.

SUMMARY

The purpose of this application is to provide a method and system for evaluating degree of eyeball movement, aiming at improving the accuracy of evaluating degree of eyeball movement and eliminating errors in the measurement process. By collecting multiple different eye position image of users in the near infrared light field, and then analyzing the collected images of multiple different eye position image, a movement angle of the degree of eyeball movement are calculated, and accurate evaluation of eyeball movement degree is achieved.

In order to achieve the above purposes, on the one hand, this application provides a method for evaluating the degree of eyeball movement, which includes the following steps:

Step S1: in a near-infrared light field of 700-1200 nm, capturing and acquiring a first eye position image of a user looking straight ahead at a position in front of the eyeball;

Step S2: capturing and acquiring a second eye position image and a third eye position image of the user's eyeball moving to a limit position in a direction to be measured in front of the eyeball; and

Step S3: comparing the first eye position image with the second eye position image and the third eye position image respectively to calculate a movement angle of the degree of eyeball movement. Furthermore, the directions to be measured may include eyeball moving upward, downward, inward, outward, inward up, outward up, inward down and outward down;

Furthermore, the Step S3 may include: adopting a convolutional neural network to segment the pupil from the images of the first eye position image, the second eye position image and the third eye position image;

taking the center $C_1$ of the first eye position image, the center $C_2$ of the second eye position image or the third eye position image, and connecting the center $C_1$ and the center $C_2$ with a straight line; locating edge points $P_1$ and $P_2$ before and after the movement of the pupil, respectively, at the same points along the straight line in both the first eye position image and either the second eye position image or the third eye position image;
connecting the edge points $P_1$ and $P_2$ to form a straight line $P_1P_2$;
dividing the straight line $P_1P_2$ into two segments, namely a straight line A and a straight line B, by a straight line perpendicular to the line $P_1P_2$ and passing through the center $C_1$;
through the eyeball radius r, obtaining a central angle $\alpha$ corresponding to the straight line A and a central angle $\beta$ corresponding to the straight line B, and obtaining the angle of eyeball movement by summing the central angle $\alpha$ and the central angle $\beta$.

Furthermore, the angle of eyeball movement is compensated based on an amplitude of pupil change, an amplitude of eyeball center displacement, and a corneal refraction error compensation derived from the first eye position image, the second eye position image or the third eye position image.

Furthermore, the angle of eyeball movement is compensated based on an amplitude of pupil change derived from the first eye position image the second eye position image or the third eye position image as follows: the position of the edge point $P_2$ is compensated by obtaining the amplitude of pupil change in the first eye position image, the second eye position image or the third eye position image.

Specifically, by calculating the amplitude of the change of the pupil diameter in the first eye position image, the second eye position image or the third eye position image along a direction perpendicular to the direction to be measured, the amplitudes can be used to compensate proportionally the position of the edge point $P_2$.

Furthermore, the angle of eyeball movement is compensated base on an amplitude of eyeball center displacement derived from the first eye position image, the second eye position image or the third eye position image, specifically: an overlapping image formed by different degrees of eyeball center displacement in the first eye position image the second eye position image or the third eye position image is obtained, and the overlapping image is translated backward to counteract the effect of the displacement on the angle of eyeball movement.

Furthermore, the angle of eyeball movement is compensated based on a corneal refraction error compensation derived from the first eye position image, the second eye position image or the third eye position image, specifically: the angle compensated by the corneal refraction error compensation is linearly related to the angle of eyeball movement.

In one aspect, the application discloses an evaluation apparatus for the degree of eyeball movement, comprising:

a first acquisition module, configured to capture and acquire a first eye position image of a user looking straight ahead in front of eyeball in a near-infrared light field of 700-1200 nm;
a second acquisition module, configured to capture and acquire a second eye position image and a third eye position image of the user's eyeball moving to a limit position in a direction to be measured in front of the eyeball; and
an image processing module, configured to compare the first eye position image with the second eye position image and the third eye position image respectively to calculate a movement angel of the degree of eyeball movement.

On another hand, the application discloses a program code stored in a computer-readable storage medium, which is loaded and executed by a processor to implement a method for evaluating the degree of eyeball movement.

Compared with the prior art, the disclosed technical solution(s) have at least the following beneficial effects: The iris and pupil can be effectively distinguished by taking eye images in near-infrared light field, so as to accurately identify the edge of pupil. By using the displacement of pupil edge points to calculate the angle of eyeball movement, the shortcomings of using an iris edge to calculate the angle of eyeball movement in the prior art are overcome.

Further, in this application, the amplitude of pupil change before and after eyeball movement, the amplitude of eye center displacement and corneal refraction error compensation are used to compensate the angle of eyeball movement, which can further improve the calculation precision of the angle of eyeball movement, so as to accurately calculate the angle of eyeball movement in each direction to be measured, and provide a more accurate judgment basis for eye detection.

DESCRIPTION OF THE DRAWINGS

In order to more clearly illustrate the technical scheme of the embodiment(s) of this application, a brief introduction of the drawings to be used in the description of the embodiment(s) of this application or the prior art is disclosed below.

Fig. 1 is a flow chart of the method for evaluating eyeball movement in this application;

Fig. 2 shows an eye image taken in visible and near-infrared light;

Fig. 3 shows an overlapping image of the second or third eye position image with the first eye position image taken in each direction to be measured;

Fig. 4 shows a calculation of the angle of eyeball movement;

Fig. 5 shows the pupil changes before and after eyeball movement;

Fig. 6 shows the overlapping images of eyeball imaging before and after eyeball movement;

Fig. 7 shows the displacement of the center of the eyeball before and after eyeball movement;

Fig. 8 shows the refraction of the corneal crystal on the pupil;

Fig. 9 shows the angle of eyeball movement calculated by using the edge of the pupil and the edge of the iris;

Fig. 10 shows a structure diagram of the apparatus for evaluating eyeball movement.

## EXAMPLES

Below, combining the attached drawings and embodiments, a further detailed description of one or more specific implementations of this application are provided. The following embodiments are used to illustrate, but not to limit, the scope of the application.

Embodiment 1:

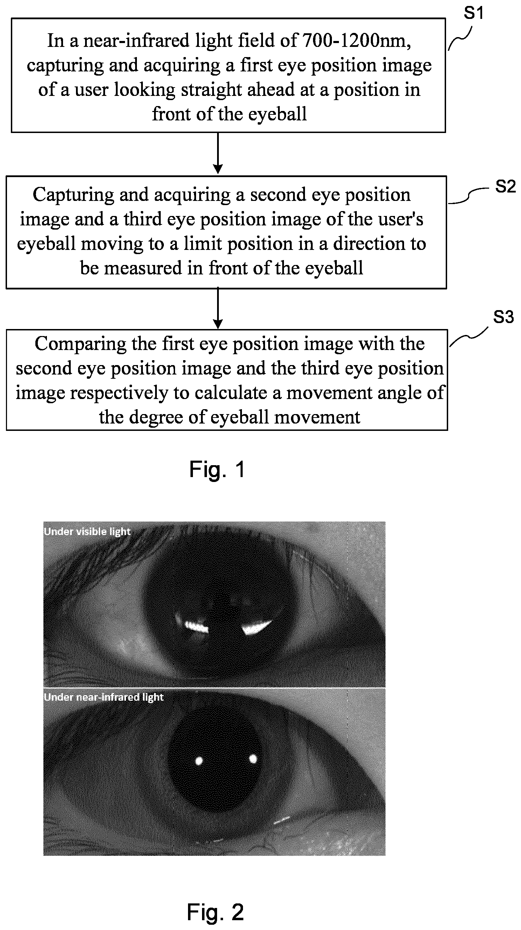

On the one hand, as shown in Fig. 1, this embodiment 1 provides a method for evaluating the degree of eyeball movement that includes the following steps:

Step S1: in a near-infrared light field of 700-1200 nm, capturing a first eye position image of a user looking straight ahead at a position in front of the eyeball;

Step S2: capturing a second eye position image and a third eye position image of the user's eyeball moving to the limit position in a direction to be measured in front of the eyeball; and

Step S3: comparing the first eye position image with the second eye position image and the third eye position image respectively to calculate a movement angle of the degree of eyeball movement. In the specific steps, when the acquisition device is used to obtain the eye image, the user's head is fixed first, such as through fixation points in the forehead rest, chin rest, and corner of the eye to achieve the user's head fixation. After fixing, the first eye position image of the user's eyes facing forward is taken in an infrared red-light field of 700-1200 nm, and the second eye position image and the third eye position image of the user's eyeball moving to the limit position in the direction indicated by the indicator light in each direction to be measured are taken.

It should be noted that the eye position refers to the position of the eyeball during the eye examination, which is divided into a first eye position, a second eye position, and a third eye position. The first eye position refers to the eye gaze position when the two eyes look straight ahead towards an infinite distance in a horizontal plane, the second eye position refers to the eye position when the eyeball moves up, down, in and out, and the third eye position refers to the eye position when the eyeball moves inwardly up, inwardly down, outwardly up and outwardly down, that is, the eye position when the eyeball moves up, under the nose, supratemporally and subtemporally. Accordingly, the first, second and third eye position images refer to the images taken by the eye at each eye gaze position, respectively.

As shown in Fig. 2, in the normal visible light band of 400-700 nm, the colors of different parts of the eye, namely the pupil, iris, and sclera, have little effect on imaging. Due to the gradual structure of the corneal limbus in the contact part of the iris and sclera, it is impossible to accurately identify the spherical center of the eyeball. The peak absorption of human melanin pigment occurs at about 335 nm, and light with wavelengths over 700 nm is almost completely not absorbed. Thus, the reflectivity of the iris is quite stable in near-infrared wavelength bands over 700 nm. Therefore, the use of near-infrared light can easily distinguish the sclera, iris and pupil boundaries. Thus, the accuracy and stability of the algorithm can be improved.

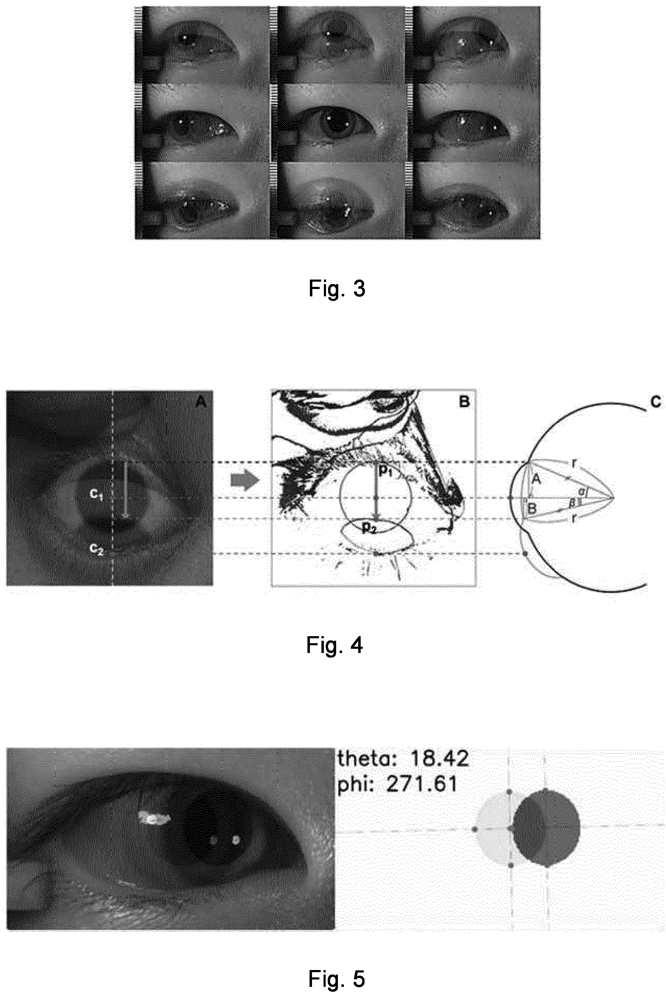

As shown in Fig. 3, the directions to be measured include eyeball moving upward, downward, inward, outward, inward up, outward up, inward down and outward down. Angles of eyeball movement of each eye in eight directions were measured. Each time one eye is measured, the other eye is shielded, and the indicator light set in each direction to be measured is used to respectively signal the left eye and the right eye to repeat the rotations in each of the aforementioned direction. The second eye position image and the third eye position image were obtained and compared respectively with the first eye position image as the benchmark, so as to calculate a movement angle of the degree of eyeball movement.

It should be noted that the above angle of eyeball movement refers to the maximum angle of rotation of the eyeball around the center of the eyeball before and after the eyeball movement.

Embodiment 2

As shown in Fig. 4, based on embodiment 1 of the above method, this embodiment 2 further defines the calculation method of the angle of eyeball movement, specifically:

adopting a convolutional neural network to segment the pupil from the images of the first eye position image, and the second eye position image when the eyeball moving downward to its limit position;

taking the center $C_1$ of the first eye position image, the center $C_2$ of the second eye position image, and connecting the center $C_1$ and the center $C_2$ with a straight line;

locating edge points $P_1$ and $P_2$ before and after movement of the pupils at the same point along the straight line in the first eye position image and the second eye position image;

connecting the edge points $P_1$ and $P_2$ to form a straight line $P_1P_2$;

dividing the line $P_1P_2$ into two segments, namely a straight line A and a straight line B, by a straight line perpendicular to the straight line $P_1P_2$ and passing through the center $C_1$;

through the eyeball radius r, obtaining a central angle $\alpha$ corresponding to the straight line A and a central angle $\beta$ corresponding to the straight line B, and obtaining the angle $\theta$ of eyeball movement by summing the central angle $\alpha$ and the central angle $\beta$.

In the above calculation method, the angle of eyeball movement before and after the eyeball movement can be obtained by calculating the center angle corresponding to the arc length of the sphere through simple analytic geometric relations. It should be noted that since the eyeball is a regular spherical structure, in each direction to be measured, such as the directions when eyeball moving inward up, inward down, outward up, and outward down, the angle of eyeball movement can be calculated by image and data processing steps after obtaining the first, second, and third eye position images.

Embodiment 3

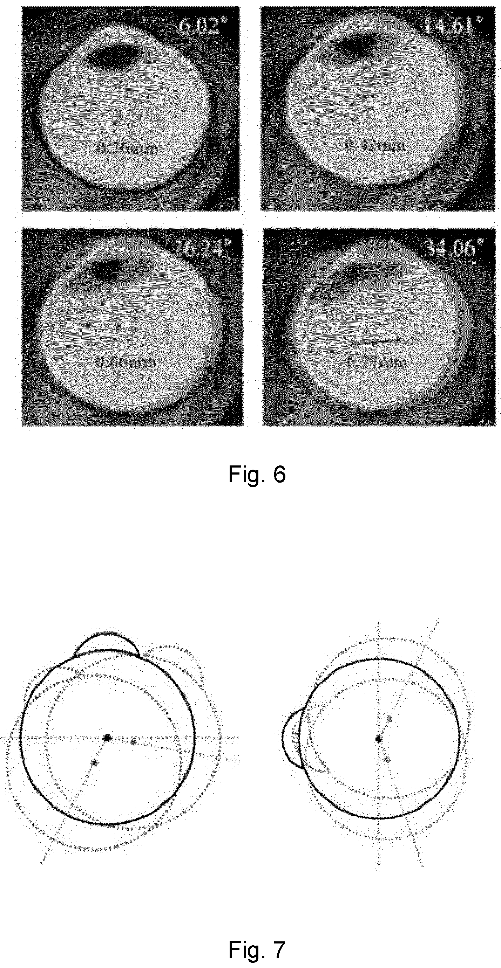

As shown in Figs. 5-7, based on the above method in embodiment 2, this embodiment 3 further defines methods of compensating the angle of eyeball movement, thereby improving the accuracy of calculating the angle of eyeball movement. In the actual measurement, it is found that the changes of the pupil before and after eyeball movement, the displacement of the center of the eyeball, and the influence of corneal refraction may cause errors in the calculation of the angle of eyeball movement. The compensation methods for the above errors are explained in detail below. First of all, as shown in Fig. 5, when the rotation of the eyeball deviates from its normal, straight-ahead position, the image of the pupil may become elliptical, and the flattening direction will be in the direction of rotation, while the image perpendicular to the direction of rotation will not be affected. So, if there is a change in the diameter of the pupil perpendicular to the direction of rotation, it comes from the change of the pupil shape. If the diameter of the pupil in orthotropia is smaller than the diameter of the pupil in strabismus, the pupil is dilated when it rotates, and if the diameter of the pupil in orthotropia is larger than the diameter of the pupil in strabismus, the pupil shrinks when it rotates. For example, if the ellipse perpendicular to the direction of rotation is shrinking, it comes from the contraction of the pupil. We connect the two centers of the separated circles to a straight line, take out the diameter of the two ellipses perpendicular to the straight line, calculate the proportion of the pupil contraction with the above two diameters, and by proportionally correcting the edge points of the ellipse used for calculation along the straight line, the position of the edge point P2 can be compensated by the amplitude of the pupil change. Thereby eliminating the influence of pupil contraction on the accuracy of calculating the angle of eyeball movement, the amplitude of the pupil change in the first eye position image, the second eye position image or the third eye position image can compensate the angle of eyeball movement. As shown in Fig. 6, when the eyeball moving, the eye is not suspended in space. Due to the surrounding soft tissues and the uneven force of the eye muscles, the eyeball rotation in different directions will cause different degrees of displacement of the eyeball center. The greater the angle of eyeball rotation, the more the center of the eye is shifted. The effect of the displacement of the center of the eyeball on the algorithm is equivalent to the misalignment of the image when overlapping alignment of emmetropic and lateral imaging, resulting in the calculated angle of eyeball movement being too large or too small. The method of compensation is to determine how much the center of the eyeball shifts, and to intentionally reverse the direction of dislocation during overlapping imaging to offset the effect of the shift.

As shown in Fig. 7, the left picture in Fig. 7 shows the changes before and after the eyeball moving in the inward and outward directions, taken from the direction of the human cranial vault, while the right picture in Fig. 7 shows the changes before and after the eyeball moving in the upward and downward directions, taken from the direction of the human cranial vault. As the eyeball moving before and after, the displacement of the center of the eyeball in the front or back direction has no effect on the image taken by the camera in front of it, it can be ignored. Only the image projected by the eyeball on the cross-section is taken. Statistically, the relationship between the direction of the eyeball movement and the displacement of the center of the eyeball on the cross-section is shown as follows:

the displacement of eyeball rotated inward by 30°: 0.69 mm inward from the center of eyeball;
the displacement of eyeball rotated outward by 30 °: 0.45mm outward from the center of eyeball;
the displacement of eyeball rotated upward by 20 °: 0.43mm downward from the center of eyeball ;
the displacement of eyeball rotated downward by 20 °: 0.43mm upward from the center of eyeball; in addition, the displacement of the eyeball center and the rotation angle of the eyeball are linear changes. If the rotation angle of the eyeball is 0°, the displacement of the eyeball center is 0 mm. The position of the center of the eyeball C2 in the calculation of the angle of eyeball movement can be corrected by the pre-statistical relationship between the above displacement and the angle, so that the angle of eyeball movement can be compensated by the magnitude of the eye center displacement in the first eye position image, the second eye position image or the third eye position image.

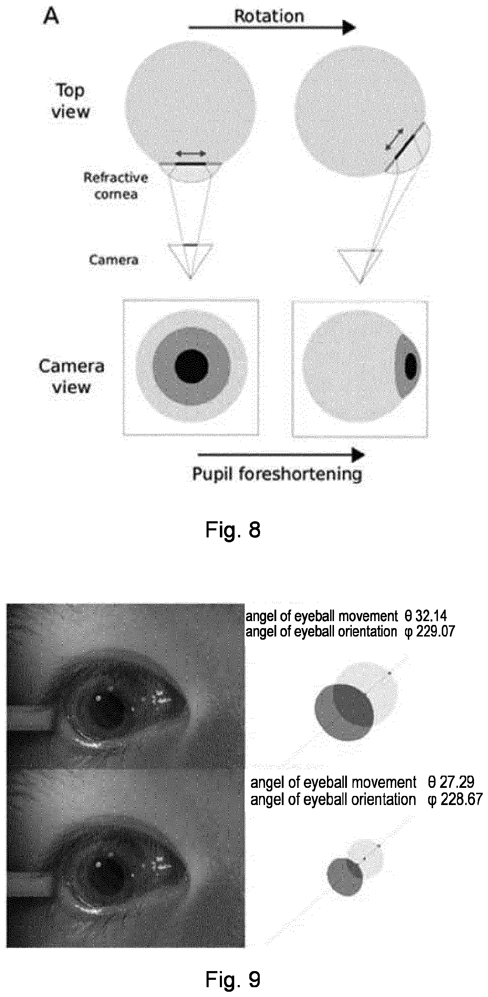

As shown in Fig. 8, when calculating the angle of eyeball movement, the edge point of the pupil is affected by the refraction of the corneal crystal, and the edge point of the pupil in the picture is not the real position, while the edge point of the iris is not affected because it does not penetrate the cornea. In the same photo, two algorithms for the pupil edge point and the iris edge point can be used to obtain different angles of eyeball movement, but the calculation using the pupil edge point has an error due to corneal refraction. Through experimental measurement, it can be seen that the difference of the angle of eyeball movement calculated by the pupil and iris is highly linear, so the formula to compensate for the corneal refraction error in the calculation of the angle of eyeball movement is as follows:

$$\text{Compensation of angle of eyeball movement } \theta = 0.13134 \times \text{angle of eyeball movement } \theta + 0.52704$$

Corrected angle of eyeball movement θ = angle of eyeball movement θ + compensation of angle of eyeball movement θ

In the above formula, the angle of eyeball movement θ is the calculated angle of eyeball movement without corneal refraction error compensation.

After taking into account the changes of the pupil before and after the above eyeball movement, the displacement of the center of the eyeball, and the influence of corneal refraction on the angle of eyeball movement and making corresponding compensation, the errors in the calculation of the angle of eyeball movement can be eliminated, and finally the accurate angle of eyeball movement can be obtained.

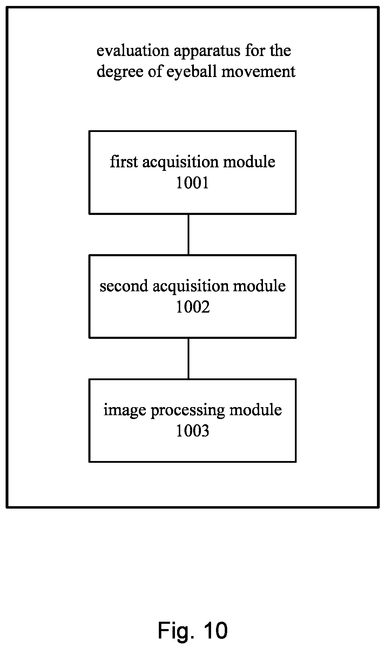

In another aspect, this application discloses an apparatus for evaluating eyeball movement, as shown in Fig. 10, including: a first acquisition module 1001, a second acquisition module 1002 and an image processing module 1003, in which:

The first acquisition module 1001 is configured to capture and acquire a first eye position image of a user looking straight ahead in front of the eyeball in a near-infrared light field of 700-1200 nm;
The second acquisition module 1002 is configured to capture and acquire a second eye position image and a third eye position image of the user's eyes moving to a limit position along a direction to be measured in front of the eyeball; and
The image processing module 1003 is configured to compare the first eye position image with the second eye position image and the third eye position image respectively, and to calculate a movement angle of the degree of eyeball movement.

On another hand, the present application discloses a computer-readable storage medium, in which at least one program code is stored, which is loaded and executed by a processor to implement the operations performed in the method of evaluating eyeball movement.

With regard to the apparatus for evaluating eyeball movement in the above embodiments, the specific ways in which each module performs operations have been described in detail in the method embodiments. Please refer to the part of the method embodiments for relevant description. In an exemplary embodiment, a computer-readable storage medium is also disclosed, including a memory storing at least one program code that is loaded and executed by the processor to perform the method of evaluating eyeball movement in the embodiments. For example, the computer-readable storage medium can be Read-Only Memory (ROM), Random Access Memory (RAM), Compact Disc Read-Only Memory (CDROM), magnetic tape, floppy disk and optical data storage devices, etc.

A person of ordinary skill in the art may understand that all or part of the steps to implement the above embodiments may be performed by hardware, or by a program in hardware related to at least one program code, which may be stored in a computer-readable storage medium, such as read-only memory, disk or optical disc, etc.

The above examples are only preferred embodiments of this application and are not intended to limit this application. Any modification, equivalent replacement, improvement, etc. made within the spirit and principles of this application shall be covered by the protection scope of this application.

**Claims**

1. A method of evaluating the degree of eyeball movement, **characterized in that** the method comprises:

    Step S1: in a near-infrared light field of 700-1200 nm, capturing and acquiring a first eye position image of a user looking straight ahead at a position in front of the eyeball;
    Step S2: capturing a second eye position image and a third eye position image of the user's eyeball moving to a limit position in a direction to be measured in front of the eyeball; and
    Step S3: comparing the first eye position image with the second eye position image and the third eye position image respectively, and calculating a movement angle of the degree of eyeball movement.

2. The method of evaluating eyeball movement in claim 1, **characterized in that** the directions to be measured includes the eyeball moving upward, downward, inward, outward, inward up, outward up, inward down and outward down.

3. The method of evaluating eyeball movement in claim 1 or 2, **characterized in that** Step S3 includes:

    adopting a convolutional neural network to segment the pupil from the images of the first eye position image, the second eye position image and the third eye position image;
    taking the center $C_1$ of the first eye position image, the center $C_2$ of the second eye position image or the third eye position image, and connecting the center $C_1$ and the center $C_2$ with a straight line;
    locating edge points $P_1$ and $P_2$ before and after the movement of the pupil, at the same point along the straight line in both the first eye position image and either the second eye position image or the third eye position image;
    connecting the edge points $P_1$ and $P_2$ to form a straight line $P_1P_2$;
    dividing the second line $P_1P_2$ into two segments, namely a straight line A and a straight line B, by a straight line perpendicular to the straight line $P_1P_2$ and passing through the center $C_1$;
    through the eyeball radius r, obtaining the central angle $\alpha$ corresponding to the straight line A and the central angle $\beta$ corresponding to the straight line B, and obtaining the angle of eyeball movement by summing the central angle $\alpha$ and the central angle $\beta$.

4. The method of evaluating eyeball movement in claim 3, **characterized in that** the angle of eyeball movement is compensated based on an amplitude of pupil change, an amplitude of eyeball center displacement, and a corneal refraction error compensation derived from the first eye position image, the second eye position image or the third eye position image.

5. The method of evaluating eyeball movement in claim 4, **characterized in that** the angle of eyeball movement is compensated based on the amplitude of pupil change derived from the first eye position image, the second eye position image or the third eye position image as follows: the position of the edge point $P_2$ is compensated by obtaining the amplitude of pupil change in the first eye position image, the second eye position image or the third eye position image.

6. The method of evaluating eyeball movement in claim 5, **characterized in that** by calculating the amplitude of the change of the pupil diameter in the first eye position image, the second eye position image or the third eye position image along the direction perpendicular to the direction to be measured, and the amplitude is used to compensate the position of the edge point $P_2$ in equal proportion.

7. The method of evaluating eyeball movement in claim 4, **characterized in that** the angle of eyeball movement is compensated based on the amplitude of the eye center displacement derived from the first eye position image, the second eye position image or the third eye position image, specifically: the overlapping image formed by different degrees of the eye center displacement in the first eye position image, the second eye position image or the third eye position image is obtained, and the overlapping image is translated backward to counteract the effect of the displacement on the angle of eyeball movement.

8. The method of evaluating eyeball movement in claim 4, **characterized in that** the angle of eyeball movement is compensated based on a corneal refraction error compensation derived from the first eye position image, the second eye position image or the third eye position image, specifically: the angle compensated by the corneal refraction error compensation is linearly related to the angle of eyeball movement.

9. A system for evaluating the degree of eyeball movement, **characterized in that** it comprises:

   a first acquisition module, configured to capture and acquire a first eye position image of a user looking straight ahead in front of eyeball in a near-infrared light field of 700-1200 nm;
   a second acquisition module, configured to capture and acquire a second eye position image and a third eye position image of the user's eyeball moving to a limit position in a direction to be measured in front of the eyeball; and
   an image processing module, configured to compare the first eye position image with the second eye position image and the third eye position image respectively to calculate a movement angle of the degree of eyeball movement.

10. A computer-readable storage medium, **characterized in that** at least one program code stored in the computer-readable storage medium, and the at least one program code is loaded and executed by a processor to implement the method of evaluating of eyeball movement as described in any of claims 1 to 8.

In a near-infrared light field of 700-1200nm, capturing and acquiring a first eye position image of a user looking straight ahead at a position in front of the eyeball | S1

Capturing and acquiring a second eye position image and a third eye position image of the user's eyeball moving to a limit position in a direction to be measured in front of the eyeball | S2

Comparing the first eye position image with the second eye position image and the third eye position image respectively to calculate a movement angle of the degree of eyeball movement | S3

Fig. 1

Fig. 2

Fig. 3

Fig. 4

Fig. 5

Fig. 6

Fig. 7

Fig. 8

angel of eyeball movement θ 32.14
angel of eyeball orientation φ 229.07

angel of eyeball movement θ 27.29
angel of eyeball orientation φ 228.67

Fig. 9

evaluation apparatus for the
degree of eyeball movement

first acquisition module
1001

second acquisition module
1002

image processing module
1003

Fig. 10

## INTERNATIONAL SEARCH REPORT

| International application No. |
|---|
| **PCT/CN2023/107803** |

**A. CLASSIFICATION OF SUBJECT MATTER**

A61B3/113(2006.01)i; A61B3/14(2006.01)i

According to International Patent Classification (IPC) or to both national classification and IPC

**B. FIELDS SEARCHED**

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNABS; CNTXT; CNKI; VEN; ENTXT; ENTXTC; WPABSC; IEEE: 佰翊医疗, 眼球, 眼睛, 眼动, 眼位, 活动, 角度, 图像, 图片, 拍摄, 拍照, 瞳孔, 补偿, 校正, eye, eyeball, movement, angle, picture, pupil, compensate, correct

**C. DOCUMENTS CONSIDERED TO BE RELEVANT**

| Category* | Citation of document, with indication, where appropriate, of the relevant passages | Relevant to claim No. |
|---|---|---|
| PX | CN 115886721 A (SHANGHAI BAIYI MEDICAL TECHNOLOGY CO., LTD.) 04 April 2023 (2023-04-04)<br>claims 1-10 | 1-10 |
| X | CN 112426123 A (NINTH PEOPLE'S HOSPITAL AFFILIATED TO SHANGHAI JIAO TONG UNIVERSITY SCHOOL OF MEDICINE) 02 March 2021 (2021-03-02)<br>description paragraphs [0004]-[0087], and figures 1A-8 | 1, 2, 9, 10 |
| X | JP 2008264341 A (UNIV CHUBE) 06 November 2008 (2008-11-06)<br>description, paragraphs [0003]-[0093], and figures 1-18 | 1, 2, 9, 10 |
| A | CN 109472189 A (TOBII AB) 15 March 2019 (2019-03-15)<br>entire document | 1-10 |
| A | CN 114391805 A (ZHONGSHAN HOSPITAL, FUDAN UNIVESITY) 26 April 2022 (2022-04-26)<br>entire document | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 October 2023** | **23 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/107803**

| Patent document cited in search report | | | Publication date (day/month/year) | Patent family member(s) | | | Publication date (day/month/year) |
|---|---|---|---|---|---|---|---|
| CN | 115886721 | A | 04 April 2023 | None | | | |
| CN | 112426123 | A | 02 March 2021 | None | | | |
| JP | 2008264341 | A | 06 November 2008 | JP | 5158842 | B2 | 06 March 2013 |
| CN | 109472189 | A | 15 March 2019 | None | | | |
| CN | 114391805 | A | 26 April 2022 | None | | | |

Form PCT/ISA/210 (patent family annex) (July 2022)

**Patent documents cited in the description**

- CN 202011260253

- CN 201710054692